(19) Europäisches Patentamt
European Patent Office
Office européen des brevets

(11) EP 1 219 297 A1

(12) **EUROPEAN PATENT APPLICATION**
published in accordance with Art. 158(3) EPC

(43) Date of publication:
**03.07.2002 Bulletin 2002/27**

(21) Application number: **00962922.1**

(22) Date of filing: **28.09.2000**

(51) Int Cl.7: **A61K 31/765**, C07D 323/00, A61P 3/00, A61P 3/04, A61P 43/00

(86) International application number:
**PCT/JP00/06704**

(87) International publication number:
**WO 01/24802 (12.04.2001 Gazette 2001/15)**

(84) Designated Contracting States:
**AT BE CH CY DE DK ES FI FR GB GR IE IT LI LU MC NL PT SE**
Designated Extension States:
**AL LT LV MK RO SI**

(30) Priority: **01.10.1999 JP 28093199**

(71) Applicant: **AMATO PHARMACEUTICAL PRODUCTS, LTD.**
**Fukuchiyama-shi, Kyoto 620-0932 (JP)**

(72) Inventors:
- **Takada, Shigeo**
  **Isehara-shi, Kanagawa 259-1112 (JP)**
- **Nagato, Yasukazu**
  **Atsugi-shi, Kanagawa 243-0122 (JP)**
- **Iwagaki, Suketsune**
  **Hadano-shi, Kanagawa 257-0028 (JP)**
- **Murakami, Masahiro**
  **Osaka 547-0026 (JP)**

(74) Representative:
**Sternagel, Fleischer, Godemeyer & Partner**
**Patentanwälte**
**An den Gärten 7**
**51491 Overath (DE)**

(54) **ANTIOBESTIC AGENTS**

(57) The object of the present invention is to provide an agent and food and drink, which have an action of suppressing excessive appetite and promoting basal metabolism and are useful for improvement and/or prevention of obesity and enhancement of the effect of kinesitherapy. The present invention provides an agent and food and drink for suppression of appetite, promotion of basal metabolism, improvement and/or prevention of obesity, or enhancement of the effect of kinesitherapy, which comprises, as an active ingredient, a mixture of cyclic and/or straight chain poly lactic acids having a condensation degree of 3 to 19.

EP 1 219 297 A1

## Description

TECHNICAL FIELD

The present invention relates to an agent and food and drink for suppressing excessive appetite, promoting basal metabolism, improving and/or preventing obesity, and/or enhancing the effect of kinesitherapy. More specifically, the present invention relates to the above-mentioned agent and food and drink, which comprise, as an active ingredient, a mixture of poly lactic acids having a condensation degree of 3 to 19.

BACKGROUND ART

Obesity is classified into primary obesity (simple obesity) and secondary obesity (symptomatic obesity). Examples of causes of primary obesity include excessive intake of energy (e.g. overeating), insufficient use of energy (e.g. lack of exercise), reduction of caloric release (basal metabolism), and the like. The majority of cases clinically diagnosed as obesity are primary obesity. Where primary obesity expresses and the obese state continues, it becomes a cause leading to various health disturbances. Secondary obesity is one resulting from some underlying disease. Examples of such secondary obesity include endocrine obesity, hypothalamic obesity, hereditary obesity, obesity caused by medicines, and the like.
Obesity is a risk factor for health, and becomes a cause for diseases such as diseases caused by an excessive burden on the circulatory system (e.g. hypertension, coronary disease, etc.), metabolic disorders (e.g. diabetes, hypertriglyceridemia, etc.), abnormality of the liver or the biliary system (e.g. cholecystolithiasis, etc.), decrease of respiratory function (e.g. hypoventilation syndrome, etc.) and diseases caused by an excessive burden on the bones or articular system (e.g. degenerative joint disease, etc.), or obesity brings about problems such as decrease of activity.
Since the principal cause for obesity is continuously excessive energy intake relative to energy consumption, correction of this state is an effective treatment for obesity. Accordingly, as a method for therapy of obesity, a combination of diet therapy (reduced diet therapy) and kinesitherapy is basically applied, and additionally, behavior therapy, psychotherapy, drug therapy, surgical treatment (e.g. surgery such as gastric reduction operation) are also applied in combination. Diet therapy is a therapeutic method for suppressing total energy intake, but it has problems such that expected weight reduction can not be achieved because of a reduced metabolic rate at rest period and that even in a successful case of weight reduction, it often centers on a reduction of lean body mass. It also has problems such that patients are subjected to physical or mental afflictions such as malnutrition oligotrophia, hunger sensation or stress. In the case of kinesitherapy, it is considered that there are effects such as improvement of metabolic rate at rest period, correction of insulin resistance and reduction of body fat, as well as an increase in consumed energy. However, kinesitherapy needs to involve aerobic exercise for 20 minutes at least three times per week, and therefore, continuous execution of this exercise is considerably difficult. Behavior therapy and psychotherapy are methods for maintaining the above-stated diet therapy and kinesitherapy, but it is generally difficult to obtain sufficient effects from these methods.
Where the above-stated therapeutic methods are not effective or where an urgent treatment is required for excessive obesity, drug therapy or surgical treatment is performed, but surgical treatment places an enormous burden on patients. Examples of therapeutic agents for obesity used for drug therapy include an appetite suppressing agent, a digestion and absorption inhibiting agent, a fat accumulation inhibiting agent and an excitometabolic agent, but these therapeutic agents for obesity have a risk of causing side effects such as drug dependence. Moreover, there is also a problem that these agents cannot be continuously used for a long-term period, since patients to whom the agents have been administered, acquire drug resistance in a short time.
Where kinesitherapy is performed, obese patients have a risk of injuring the joints of the lower extremities if intensive exercise is imposed thereon, and therefore it is generally preferable to perform light swimming. Thus, there is considered, as a preferred method for improvement of obesity, a method for increasing muscles by a combined use of an agent for activating energy metabolism with few side effects, with light exercises such as light swimming so as to promote energy consumption by increasing basal metabolism.
Hence, there has been a desire for development of an effective agent for improvement of obesity, which can continuously be used for a long-term period without placing a burden on patients, and with fewer side effects.

DISCLOSURE OF THE INVENTION

An object of the present invention to be achieved is to provide an agent, which has an action of suppressing excessive appetite and promoting basal metabolism and is useful for improvement and/or prevention of obesity and enhancement of the effect of kinesitherapy. Moreover, another object of the present invention to be achieved is to provide food and drink, which suppress excessive appetite and promote basal metabolism and are useful for improvement and/or prevention of obesity, and enhancement of the effect of kinesitherapy.

As a result of focused research to achieve the aforementioned objects, the present inventors have found that administration of a mixture of cyclic and/or straight chain poly lactic acids having a condensation degree of 3 to 19 to obese mice, enables suppression of excessive appetite thereof, promote basal metabolism thereof and reduce body weight thereof, thereby completing the present invention. From studies that have been made so far, it has been reported that a mixture of cyclic and/or straight chain poly L-lactic acids having a condensation degree of 3 to 19 is useful as an antineoplastic agent (Japanese Patent Application Laying-Open (Kokai) Nos. 9-227388 and 10-130153) and also as a QOL improving agent for cancer patients (Japanese Patent Application No. 11-39894 Specification; Bulletin of Japan Society of Clinical Oncology, Vol. 33, No. 3, p. 493). Furthermore, it has also been found that the mixture of cyclic and/or straight chain poly lactic acids having a condensation degree of 3 to 19 has a hypoglycemic action and is useful as a medicament for prevention and/or treatment of diabetes or diabetes complications (Japanese Patent Application No. 11-224883), but it has been found for the first time by the present inventors that this mixture of poly lactic acids is useful for suppression of excessive appetite, promotion of basal metabolism, and improvement and/or prevention of obesity.

Thus, according to the first aspect of the present invention, there is provided an appetite suppressing agent which comprises, as an active ingredient, a mixture of cyclic and/or straight chain poly lactic acids having a condensation degree of 3 to 19.

According to the second aspect of the present invention, there is provided a basal metabolism promoting agent which comprises, as an active ingredient, a mixture of cyclic and/or straight chain poly lactic acids having a condensation degree of 3 to 19.

According to the third aspect of the present invention, there is provided an agent for improving and/or preventing obesity which comprises, as an active ingredient, a mixture of cyclic and/or straight chain poly lactic acids having a condensation degree of 3 to 19.

According to the fourth aspect of the present invention, there is provided an agent for enhancing the effect of kinesitherapy that comprises, as an active ingredient, a mixture of cyclic and/or straight chain poly lactic acids having a condensation degree of 3 to 19.

In the present invention, preferably the lactic acid that is a repeating unit in the poly lactic acid consists substantially of L-lactic acid.

Preferably, the mixture of cyclic and/or straight chain poly lactic acids having a condensation degree of 3 to 19 that is used in the present invention is a fraction obtained by condensing lactic acids by dehydration under an inactive atmosphere, subjecting the ethanol- and methanol-soluble fractions of the obtained reaction solution to reverse phase column chromatography, and eluting with 25 to 50 weight % acetonitrile aqueous solution of pH 2 to 3 and then with 90 weight % or more acetonitrile aqueous solution of pH 2 to 3.

Preferably, condensation by dehydration is performed by stepwise decompression and temperature rise under nitrogen gas atmosphere.

Preferably, reverse phase column chromatography is performed by ODS column chromatography.

According to another aspect of the present invention, there are provided food and drink for suppression of appetite, promotion of basal metabolism, improvement and/or prevention of obesity, and/or enhancement of the effect of kinesitherapy, which comprises, as an active ingredient, a mixture of cyclic and/or straight chain poly lactic acids having a condensation degree of 3 to 19.

According to still another aspect of the present invention, there is provided the use of a mixture of cyclic and/or straight chain poly lactic acids having a condensation degree of 3 to 19 in the production of an appetite suppressing agent, a basal metabolism promoting agent, an agent for improving and/or preventing obesity or an agent for enhancing the effect of kinesitherapy, and food and drink for suppression of appetite, promotion of basal metabolism, improvement and/or prevention of obesity, and/or enhancement of the effect of kinesitherapy.

According to a still further aspect of the present invention, there is provided a method for suppression of excessive appetite, method for promotion of basal metabolism, method for improving and/or preventing obesity, or method for enhancement of the effect of kinesitherapy, which comprises a step of administering an effective amount of a mixture of cyclic and/or straight chain poly lactic acids having a condensation degree of 3 to 19 to mammals such as humans.

BRIEF DESCRIPTION OF THE DRAWINGS

Figure 1 shows a mass spectrum of the mixture of poly lactic acids obtained by Production Example 1 of the present specification.

THE BEST MODE FOR CARRYING OUT THE INVENTION

The embodiments and methods for carrying out the present invention are described in detail below.

The term "the agent of the present invention" in the present specification is used to include each of an appetite suppressing agent, a basal metabolism promoting agent, an agent for improving and/or preventing obesity and an agent for enhancing the effect of kinesitherapy.

The term "an appetite suppressing agent" in the present specification is used to broadly mean an agent administered for the purpose of reducing appetite, and examples of such agents include an agent for the purpose of improving and/or preventing obesity, or treating hyperphagia, but are not limited thereto.

The term "a basal metabolism promoting agent" in the present specification is used to broadly mean an agent administered for the purpose of promoting basal metabolism in the body and activating energy metabolism, and examples of such agents include an agent for the purpose of improving and/or preventing obesity, but are not limited thereto.

A factor responsible for the usefulness of the agent of the present invention for improving and/or preventing obesity is that the agent is based on an appetite suppressing action and a basal metabolism promoting action. However, the agent for improving and/or preventing obesity of the present invention is not limited to one which depends on such an appetite suppressing action or basal metabolism promoting action, and an action of improving and/or preventing obesity due to other mechanism of action are not excluded therefrom.

The term "obesity" in the present specification is used to mean obesity in the broadest sense, and this is a concept that includes the more strict sense of "adiposis". Among cases diagnosed as obesity, "adiposis" generally refers to a pathological state, which has complications caused by obesity or wherein onset of such complications are predicted unless weight reduction is performed, and means the state where weight reduction treatment is medically required.

The term "obesity" in the present specification generally means overweight, and more specifically means a state where fat tissues excessively accumulate in the body. In recent years, in respect of accumulation of subcutaneous fat, offal fat, etc., it has been reported that the onset of adult diseases such as hypertension, hyperlipidemia and diabetes is associated with splanchnic fat type obesity wherein fat accumulates among organs, rather than subcutaneous fat type obesity wherein fat accumulates in subcutaneous tissues. It can be expected that the agent or food and drink of the present invention have an effect of preventing these diseases.

Methods for judging obesity are not particularly limited, but for example, there is a method involving the use of BMI (body mass index) as a scale, which is used throughout the world. BMI is a value obtained by dividing body weight (kg) by the square of body height (m). Using BMI, calculation of a standard body weight (ideal weight) of Japanese adults from the following formula, has been proposed.

$$\text{Standard body weight (kg)} = \text{body height (m)}^2 \times 22$$

There is a method for judging the state where measured body weight exceeds 120% of standard body weight (calculated value) as obesity, but this method is only an example of methods for judging obesity, and is not intended to limit the scope of the present invention. Since standard body weight (ideal body weight) is different among individuals, depending on sex, age or difference in life habits, judgment of obesity is also different among individuals.

The agent or food and drink of the present invention can be used for enhancement of the effect of kinesitherapy. The term "kinesitherapy" in the present specification broadly means therapy including the performance of exercises to consume redundant energy. Diseases or physical states that are the targets of treatment and/or prevention are not particularly limited. Kinesitherapy can be performed for treatment and/or prevention of, for example, obesity, diabetes and so on. The effect of such kinesitherapy can be enhanced by ingesting the agent or food and drink of the present invention. The period for ingestion of the agent or food and drink of the present invention may be before, during or after performing kinesitherapy, and is not particularly limited.

In the agent and food and drink according to the present invention, a mixture of cyclic and/or straight chain poly lactic acids having a condensation degree of 3 to 19 is used as an active ingredient.

The term "a mixture of poly lactic acids" used in the present invention means a mixture wherein cyclic and/or straight chain poly lactic acids having a condensation degree of 3 to 19 are present at any ratio. That is to say, the term "mixture" does not only mean a mixture of poly lactic acids having any condensation degree ranging from 3 to 19, but is also used as a concept including a mixture of cyclic and straight chain poly lactic acids. As is described below in the present specification, "a mixture of poly lactic acids" can be obtained by condensing lactic acids by dehydration and then performing purification by a suitable method. Although the term "a mixture of poly lactic acids" is used in the present specification for the sake of convenience, this term also includes a poly lactic acid consisting of a single ingredient such as a cyclic poly lactic acid having single condensation degree or a straight chain poly lactic acid having

single condensation degree.

The term "condensation degree" is used to mean the number of lactic acid unit that is a repeating unit in poly lactic acids. For example, the cyclic poly lactic acid is assumed to have the following structural formula wherein n represents condensation degree (n = 3 to 19).

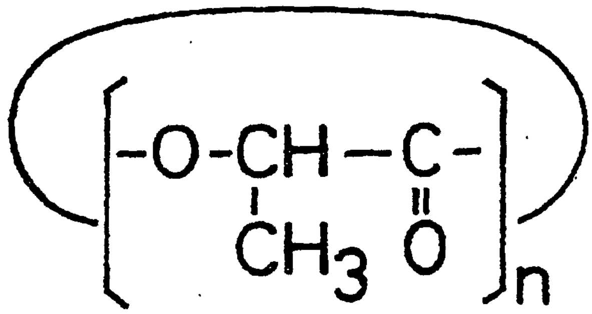

When "lactic acid" is simply referred to in the present specification, this lactic acid includes all of L-lactic acid, D-lactic acid or a mixture comprising these types of lactic acid at any ratio. Preferably in the present invention, the lactic acid consists substantially of L-lactic acid. The term "substantially" is used herein to mean that the ratio of L-lactic acid units in a mixture of poly lactic acids (number of L-lactic acid unit / number of L-lactic acid unit + number of D-lactic acid unit × 100) is, for example, 70% or more, preferably 80% or more, more preferably 85% or more, further more preferably 90% or more, and particularly preferably 95% or more. The ratio of L-lactic acid units in a mixture of poly lactic acids depends on the ratio of L-lactic acid and D-lactic acid that exist in lactic acids used as a starting substance.

The methods for producing a mixture of cyclic and/or straight chain poly lactic acids having a condensation degree of 3 to 19 are not particularly limited, and the mixture of poly lactic acids can be obtained by the production methods described, for example, in Japanese Patent Application Laying-Open (Kokai) Nos. 9-227388 and 10-130153 or Japanese Patent Application No. 11-39894 (All publications cited herein are incorporated herein by reference in their entirety).

More specifically, for example, a mixture of cyclic and/or straight chain poly lactic acids having a condensation degree of 3 to 19 can be obtained by the following method A.

Method A:

First, lactic acid (preferably, lactic acid substantially consisting of L-lactic acid) is condensed by dehydration under an inactive atmosphere. Examples of the inactive atmosphere include nitrogen gas and argon gas, and nitrogen gas is preferred.

Dehydration and condensation reaction is carried out at a temperature of 110°C to 210°C, preferably 130°C to 190°C under normal pressure to reduced pressure of approximately 1mmHg, and particularly preferably the reaction is carried out by stepwise decompression and stepwise temperature rise. A reaction period can be determined as appropriate. For example, the reaction can be carried out for 1 to 20 hours. Where stepwise decompression and stepwise temperature rise are applied, reaction is performed by dividing the reaction period into two or more partial reaction periods, and then determining pressure and temperature for each of the reaction periods. Where stepwise decompression is applied, pressure can be reduced, for example, from a normal pressure to 150mmHg and then to 3mmHg. Where stepwise temperature rise is applied, temperature can be raised, for example, from 145°C to 155°C and then to 185°C. Practically, the reaction can be carried out by using these conditions in combination, for example, 145°C, normal pressure, 3 hours; 145°C, 150mmHg, 3 hours; 155°C, 3mmHg, 3 hours; and 185°C, 3mmHg, 1.5 hours.

Subsequently, ethanol and methanol are added to the reaction mixture obtained by the dehydration and condensation reaction, and the mixture is filtered. The obtained filtrate is dried to obtain ethanol- and methanol-soluble fractions. The term "ethanol- and methanol-soluble fractions" is used in the present specification to mean fractions soluble in a mixed solution of ethanol and methanol. In order to obtain ethanol and methanol-soluble fractions, a reaction mixture obtained by dehydration and condensation reaction is mixed with ethanol and methanol, where the ratio of ethanol and methanol can be determined as appropriate. For example, the ratio is ethanol:methanol = 1 : 9. The order, method and the like for adding ethanol and methanol to a reaction mixture are not limited, and may be selected as appropriate. For example, ethanol may be added at first to the reaction mixture obtained by the dehydration and condensation reaction, and then methanol may be added thereto.

The thus obtained ethanol- and methanol-soluble fractions are subjected to reverse phase column chromatography, especially to chromatography where an octadecylsilane (ODS) column is used. First, fractions eluted with 25 to 50 weight % acetonitrile aqueous solution of pH 2 to 3 are removed, and then fractions eluted with 90 weight % or more acetonitrile aqueous solution of pH 2 to 3, preferably 99 weight % or more acetonitrile aqueous solution, are collected so as to obtain a mixture of cyclic and/or straight chain poly lactic acids having a condensation degree of 3 to 20.

The thus obtained mixture of cyclic and/or straight chain poly lactic acids is neutralized with an alkaline substance such as sodium hydroxide, and is dried under reduced pressure, and then according to standard techniques, the mixture can be formulated in a desired form as mentioned below.

Other examples of the methods for producing a mixture of cyclic and/or straight chain poly lactic acids having a condensation degree of 3 to 20 used in the present invention include a method described in Japanese Patent Application No. 11-265715 (hereinafter referred to as method B), or a method described in Japanese Patent Application No. 11-265732 (hereinafter referred to as method C) (All publications cited herein are incorporated herein by reference in their entirety). Methods B and C will be described specifically below.

Method B:

Method B is a method for producing a cyclic lactic acid oligomer which comprises polymerizing lactid (3,6-dimethyl-1,4-dioxane-2,5-dione) in the presence of an alkali metal compound represented by RYMe [wherein R represents an aliphatic group, aromatic group, substituted or unsubstituted silyl group, or lactamide group ($-CH(CH_3)CONH_2$ group), Y represents oxygen atom, sulfur atom, or NR', in which R' represents hydrogen atom, aliphatic group or aromatic group, and Me represents alkali metal].

An aliphatic carbohydrate group in the present specification may be straight chain, branched-chain, cyclic, or any combination of these, and may be a saturated or unsaturated group. The carbon number of the aliphatic carbohydrate group is 1 to 12, preferably 1 to 6. Examples of the aliphatic carbohydrate group include a chain (including both straight chain and branched-chain) alkyl group such as methyl, ethyl, propyl, butyl, octyl and dodecyl; and a cycloalkyl group (for example, cyclopropyl, cyclobutyl, cyclopentyl, and cyclohexyl).

An aromatic carbohydrate group in the present specification includes an aryl group which may have a substituted group such as an alkyl group, and an arylalkyl group, and the carbon number thereof is 6 to 12, preferably 6 to 10. Examples of the aryl group which may have a substituted group such as an alkyl group include phenyl, tolyl, and naphthyl. Examples of the arylalkyl group include benzyl, phenethyl, and naphthylmethyl.

Examples of a substituted group of a substituted or unsubstituted silyl group include an aliphatic carbohydrate group or aromatic carbohydrate group. Specific examples of the substituted silyl group include a trimethylsilyl group, triphenylsilyl group or t-butyldimethylsilyl group.

Examples of alkali metal represented by Me include lithium, sodium or potassium, and lithium is preferred.

An alkali metal compound represented by RYMe can be obtained by allowing alkyl-alkali metal, such as n-butyl lithium, to react with R'-YH (wherein R' represents an aliphatic carbohydrate group or aromatic carbohydrate group, and Y represents oxygen atom or sulfur atom).

Specifically, reaction can be carried out by preparing a solution by dissolving an alcohol compound or thiol compound represented by R'-YH in an appropriate solvent (for example, an ether-based solvent, such as anhydrous tetrahydrofuran or anhydrous diethyl ether), adding alkyl-alkali metal, such as n-butyl lithium, to the solution in an amount almost equivalent to that of the alcohol compound or thiol compound, and then stirring the solution.

Reaction may be carried out at a low temperature (for example, -78°C) for several minutes to 1 hour.

When a cyclic lactic acid oligomer used in the present invention is produced by allowing lactid (3,6-dimethyl-1,4-dioxane-2,5-dione) to react in the presence of an alkali metal compound (RYMe), the cyclic lactic acid oligomer can be produced by adding a lactid solution in an appropriate solvent (for example, anhydrous tetrahydrofuran) to a reaction mixture containing the alkali metal compound obtained as described above, and stirring the solution.

The molar ratio of the amounts of the alkali metal compound (RYMe) and lactid is 1:1 to 1:10, preferably about 1:2 to 1:5, for example, 1:3 or 1:4.

A reaction temperature range is -78°C to room temperature. Reaction is preferably carried out by starting from a temperature of -78°C, and gradually raising it to room temperature. In addition, a reaction pressure is not specifically limited, and normal pressure is preferred.

As described above, the reaction is preferably carried out in the presence of a solvent. A preferred reaction solvent is a solvent inactive in reaction. For example, an ether-based solvent (anhydrous tetrahydrofuran, anhydrous diethylether, or the like) can be used. Reaction is preferably performed under an inactive gas atmosphere such as nitrogen gas and argon gas.

The mechanism of the above-described reaction for the synthesis of the cyclic lactic acid oligomer used in

the present invention is described further below. However, the present invention is not restricted by this theory, and a cyclic lactic acid oligomer which is synthesized in a reaction with a mechanism differing from the mechanism below may be used in the present invention.

In the above reaction (herein below, an example is described wherein alkali metal is Li ), first a lithium compound is reacted with lactid so that a derivative of chain lactic acids which is represented by the following formula is synthesized:

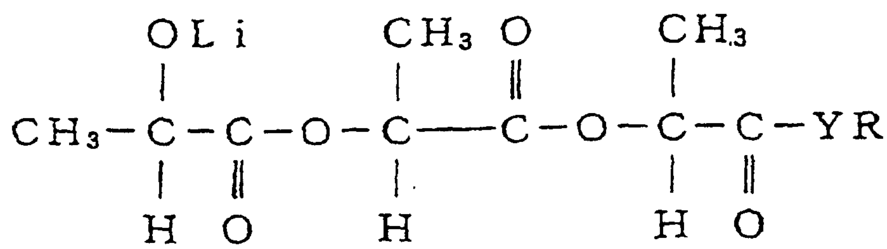

(wherein Y and R have the same meaning as described above) .
Then, lactid is reacted with this compound, so that a chain lactic acid oligomer which is represented by the following formula is synthesized:

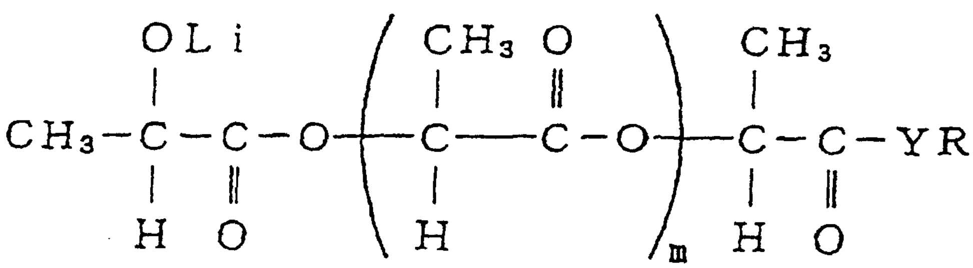

(wherein m represents a number from 1 to 21, and Y and R have the same meaning as described above). Then RYLi leaves from the compound, which is followed by cyclization. Thus the cyclic lactic acid oligomer of the above formula (1) is synthesized.

The composition (that is, the mixing ratio of a cyclic lactic acid oligomer and a chain lactic acid oligomer) of the lactic acid oligomer obtained as described above fluctuates depending on the alkali metal compound used as a reaction assistant. Where an alkali metal compound of alkyl alcohol having a carbon number of 1 to 3 (ROMe) (wherein R represents an alkyl group with carbon number 1 to 3, and Me represents alkali metal) is used as an alkali metal compound, a mixture of a cyclic lactic acid oligomer and a chain oligomer (proportion of the cyclic lactic acid oligomer: 80 to 85 weight %) is obtained. When a lithium compound of alkyl alcohol having a carbon number of 4 or more such as t-butyl alcohol, or thiophenol compound is used as an alkali metal compound, substantially only a cyclic lactic acid oligomer can be selectively obtained.

The polymerization degree of the cyclic lactic acid oligomer used in the present invention is 3 to 20, preferably 3 to 17. This polymerization degree fluctuates depending on the type of alkali metal compound to be used, reaction temperature and reaction period.

Further, a mixture of cyclic (and chain, depending on the case) lactic acid oligomers with different polymerization degrees is thought to be present in the reaction product resulting from polymerization reaction of lactid in the presence of the above alkali metal compound. In the present invention, a mixture comprising lactic acid oligomers with different polymerization degrees can be used. However, a single lactic acid oligomer having a single polymerization degree may be obtained by purifying with a technique (for example, gel filtration, HPLC or the like) appropriate for separating compounds of differing molecular weights from a reaction mixture containing the above lactic acid oligomers with different polymerization degrees, and may be used herein.

In the above method of producing a cyclic lactic acid oligomer, substantially only cyclic lactic acid oligomers can also be selectively obtained by carrying out reaction in the same manner as described above with the exception that an alkali metal compound of lactamide (in particular, a lithium compound) (that is, a compound wherein R represents

$-CH(CH_3)CONH_2$ group) is used as an alkali metal compound.

Method C:

This method comprises:

(i) a first heating step which comprises heating lactic acid under a pressure condition of 350 to 400 mmHg and to a temperature of 120 to 140°C so as to perform dehydration and condensation, and distilling off and removing only by-product water without distilling lactid off;
(ii) a second heating step for synthesizing a product condensed by dehydration comprising chain lactic acid oligomers as the main ingredient, which comprises, after completion of the first heating step, heating the reaction product to a temperature of 150 to 160°C while reducing the reaction pressure to 15 to 20 mmHg at a decompression rate of 0.5 to 1 mmHg/min, wherein only by-product water is distilled off and removed while avoiding distillation of lactid; and after the reaction pressure is reduced to 15 to 20 mmHg, maintaining the reaction under the same pressure condition and at a reaction temperature of 150 to 160°C;
(iii) a third heating step for synthesizing cyclic oligomers which comprises, after completion of the second heating step, heating under a pressure condition of 0.1 to 3 mmHg and at 150 to 160°C to cyclize the chain lactic oligomer.

In this method, first, in the first heating step, lactic acid is heated under reduced pressure to perform dehydration and compression reaction. In this case the reaction period is 3 to 12 hours, preferably 5 to 6 hours. To allow the reaction in the first heating step to proceed smoothly, by-product water produced by condensation of lactic acids by dehydration is distilled off. At this time, distillation of by-product water is performed such that lactid, which is the dehydrated condensed product of two molecules of lactic acid, is not distilled off. To achieve such purpose, the reaction pressure is maintained at a reduced pressure, preferably 300 to 500 mmHg, more preferably 350 to 400 mmHg. Under this pressure condition, heating is performed at a temperature range of 100 to 140°C, preferably 130 to 140°C. The reaction product produced by reaction in the first heating step mainly comprises as the main ingredient a dehydrated condensed product of 3 to 23 molecules of lactic acid.
To obtain oligomers having an increased average degree of polymerization in the second heating step after completion of the above first heating step, heating is performed at a temperature higher than the reaction temperature of the above first heating step, preferably at 145°C to 180°C, more preferably 150°C to 160°C, while the reaction pressure is reduced to 10 to 50 mmHg, preferably 15 to 20 mmHg, so that dehydration and condensation reaction is further continued.
As with the reaction in the above first heating step, reaction is performed under a condition where by-product water, but not lactid, is distilled off, to allow the reaction to proceed smoothly. The rate at which reaction pressure is reduced to a pressure in the above range (decompression rate) is normally required to be maintained within a range of 0.25 to 5 mmHg/min, preferably 0.5 to 1 mmHg/min, in order to avoid distillation of lactid and increase the reaction efficiency. A decompression rate lower than the above range is not preferred because it will increase the time required to reduce pressure to a given pressure. On the other hand, a decompression rate higher than the above range is also not preferred because it will cause lactid to be distilled off together with by-product water.
After the reaction pressure is reduced to a certain pressure, reaction is further continued at that reaction pressure. The heating time period in this case is 3 to 12 hours, preferably 5 to 6 hours.
A lactic acid oligomer having an average polymerization degree of 3 to 30, preferably 3 to 23 is obtained by the reaction in the above second heating step. The proportion of cyclic oligomers in the oligomers in this case is normally about 70 to 80 weight %.
In the third heating step, after completion of the above second heating step, a reaction pressure is maintained at 0.25 to 5 mmHg, preferably 0.5 to 1 mmHg, and reaction is further continued at a temperature of 145 to 180°C, preferably 150 to 160°C. A reaction period is 3 to 12 hours, preferably 5 to 6 hours. By-product water produced in this case is also distilled off. In this case, distillation of lactid is preferably avoided. However, since the reaction product contains almost no lactid, it is not required to specially lower the decompression rate.
Lactic acid oligomers produced by reaction in the above third heating step have an average polymerization degree of 3 to 30, preferably 3 to 23, and contain cyclic oligomer in the proportion of 90 weight % or more, preferably 99 weight % or more.
The above methods A, B and C merely show some of specific examples of methods of producing a mixture of poly lactic acids used in the present invention. A mixture of poly lactic acids which is produced by other methods can also be used in the present invention.
The dosage form of the agent of the present invention is not particularly limited, and any form suitable for the purpose of therapy or prevention can be selected from dosage forms for oral or parenteral administration. Examples of dosage forms suitable for oral administration include a tablet, a capsule, a powder, a granule, a parvule, a syrup, a

solution, an emulsion, a suspension, a chewable tablet, and the like. Examples of dosage forms suitable for parenteral administration include, but are not limited to, transcutaneous absorbing agents in the form of an injection (e.g. a subcutaneous, intramuscular or intravenous injection, and the like), a drop, an inhalant, a nebula, a suppository, a gel, ointment or the like, and transcutaneous absorbing agents in the form of a trans-mucous absorbing agent, a patch agent, a tape agent or the like. Dosage forms for oral administration is preferred.

Liquid formulations suitable for oral administration such as a solution, emulsion or syrup can be produced using water; sugars such as sucrose, sorbit or fructose; glycols such as polyethylene glycol or propylene glycol; oils such as sesame oil, olive oil or soybean oil; antiseptics such as p-hydroxybenzoate; and flavors such as strawberry flavor and peppermint. In order to produce solid formulations such as capsule, tablet, powder or granule, there can be used an excipient such as lactose, glucose, sucrose or mannite; a disintegrator such as starch or sodium alginate; a lubricant such as magnesium stearate or talc; a binder such as polyvinyl alcohol, hydroxypropylcellulose or gelatin; a surfactant such as fatty acid ester; and a plasticizer such as glycerine, and the like.

Formulations for an injection or drop that are suitable for parenteral administration preferably comprise, as an active ingredient, the above substance in a dissolved or suspended state in a sterilized aqueous medium which is isotonic to the recipient's blood. For example, in the case of an injection, a solution can be prepared using an aqueous medium consisting of a saline solution, a glucose solution or a mixture of a saline solution and a glucose solution. In the case of a formulation for intestinal administration, it can be prepared using carriers such as theobroma oil, hydrogenated lipids or hydrogenated carboxylic acid, and can be provided as a suppository. In order to produce a nebula, the above substance as an active ingredient may be dispersed as microparticles, and a carrier which does not irritate the recipient's cavitas oris and respiratory tract mucosa and which facilitates absorption of the active ingredient can be used. Specific examples of carriers include lactose, glycerine, and the like. Formulations having a form such as aerosol or dry powder may be prepared depending on the properties of the substance of an active ingredient and the carrier to be used. One or two or more auxiliary ingredients selected from glycols, oils, flavors, an antiseptic, an excipient, a disintegrator, a lubricant, a binder, a surfactant, a plasticizer and the like may be added to these formulations for parenteral administration.

The dose and dosage frequency of the agent of the present invention are determined as appropriate, depending on various factors such as type and severity of the disease, dosage purpose, dosage form, condition such as age or body weight of a patient, and the presence or absence of complication. Generally, the dose of an active ingredient per day is 10 to 2,000 mg/kg, preferably 10 to 200 m/kg, and more preferably 50 to 150 mg/kg. It is preferred that the above dose of formulation is dividedly applied about once to 4 times per day, preferably about twice to 4 times per day.

The agent of the present invention can be administered to any mammal including humans.

The mixture of cyclic and/or straight chain poly lactic acids having a condensation degree of 3 to 19 used in the present invention is used not only as a medicament, but also may be mixed into a drinkable preparation, such as a nutrition supplement drinkable preparation, or can be mixed into health food as a food additive. Specific examples of food and drink according to the present invention which comprises the mixture of cyclic and/or straight chain poly lactic acids having a condensation degree of 3 to 19, include health foods or supplements including drinks, such as those generally called a soft drink, drinkable preparation, health food, specified supplement food, functional food, function-activating food, nutritional supplementary food, supplement, feed, feed additive and the like.

Specific examples of food and drink into which the mixture of poly lactic acids can be mixed include confectionary, such as a chewing gum, chocolate, candy, sweet tablet, jelly, cookie, biscuit, and yogurt; frozen deserts, such as ice cream and sherbet; beverages, such as tea, soft drink (including juice, coffee, cocoa and the like), nutrition supplement drinkable preparation, and cosmetic drinkable preparation; and all other food and drink, such as bread, ham, soup, jam, spaghetti, and frozen food. Alternatively, the mixture of poly lactic acids used in the present invention can also be used by adding to seasoning, food additives, and the like.

By the use of the above food and drink of the present invention, there can be provided safe food and drink which can exert the effect of shape-up or diet while substantially showing no toxic side effect.

The food and drink of the present invention encompasses food and drink in every form, and the types are not specifically limited. That is, the food and drink can be provided by mixing the mixture of cyclic and/or straight chain poly lactic acids having a condensation degree of 3 to 19 into the above-mentioned various food and drink, or various nutrient compositions, such as various oral or enteral nutrient preparations or drinks. Compositions of such food and drink may include protein, lipid, carbohydrate, vitamin and/or mineral, in addition to the mixture of cyclic and/or straight chain poly lactic acids having a condensation degree of 3 to 19. The form of the food and drink is not specifically limited, and may be in any form, such as solid, powdery, liquid, gel, and slurry forms, so far as it is in a form that is easily ingested.

The content of the mixture of poly lactic acids in the food and drink is not specifically limited, and is generally 0.1 to 20 weight %, more preferably approximately 0.1 to 10 weight %.

The mixture of poly lactic acids is preferably contained in the food and drink in an amount which achieve the object of the present invention. Preferably, about 0.1 g to 10 g, more preferably about 0.5 g to 3 g, of the mixture of poly lactic acids is contained per food or drink to be ingested.

The present invention is further described in the following examples, but the scope of the present invention is not limited by the examples in any way.

EXAMPLES

Production Example 1: Production of a mixture of poly lactic acids

500 ml of L-lactic acid (to which D-lactic acid was also mixed) was placed into a separable flask in a mantle heater. 300ml/min of nitrogen gas was flowed therein while stirring. Accumulated water was introduced into a flask equipped with a reflux condenser via a warmed descending type connecting tube, while heating at 145°C for 3 hours. Furthermore, after pressure was reduced to 150 mmHg and heated at the same temperature for 3 hours, the mixture was heated at 155°C for 3 hours under a reduced pressure of 3 mmHg, and then at 185°C for 1.5 hours under a reduced pressure of 3 mmHg to obtain poly lactic acids as a reaction product.

The obtained poly lactic acids were kept at 100°C, and 100ml of ethanol and 400ml of methanol were separately added thereto, and then the mixture was allowed to be cooled. This mixture was added to 500ml of methanol, and the mixture was well stirred and left to stand. Then, the mixture was filtrated for purification. The filtrate was subjected to vacuum drying and then dissolved in acetonitrile to obtain 200ml (stock solution) in total.

The stock solution was subjected to a reverse phase ODS column (TSK gel ODS-80 TM) which was previously equilibrated, and was stepwise eluted with 30%, 50% and 100% acetonitrile (pH2.0) each containing 0.01M hydrochloric acid to obtain poly lactic acids (condensation degree of 3 to 19) as an acetonitrile 100% elution fraction. The mass spectrum of the obtained substance is shown in Fig. 1. As is clear from the regular fragment ion peaks in Fig. 1, the obtained mixture of poly lactic acids mainly comprises cyclic condensate, and a small amount of linear condensate is contained therein.

Test Example 1: Effect of a mixture of poly lactic acids on food consumption of obese mice

Obese mice (genetic hyperphagia mice (type II diabetes mice), C57BL/KsJ-db/db Jcl, 6-week-old females, CLEA Japan, Inc.) were divided into three groups: (1) a CE2EX group (12 mice), which were fed with standard solid food CE2 (obtained from CLEA Japan, Inc.) and were subjected to swimming for 20 minutes every other day; (2) a CPL group (12 mice), which were fed with standard solid food CE2 containing 1 weight % (where a fraction obtained by chromatography is used, and its concentration is 1 weight %) of a mixture of poly lactic acids (hereinafter, also referred to as CPL); and (3) a CPLEX group (12 mice), which were fed with the same solid food as used for the CPL group and were subjected to swimming for 20 minutes every other day, and then the food consumption per day was observed for each group.

Since the food consumption of the group in which mice were fed with standard solid food CE2 and were not subjected to swimming, was almost the same as the food consumption of the CE2EX group, the data of this group was omitted. The results are shown in the following Table 1.

As is clear from the results shown in Table 1, the food consumption of the CPL and CPLEX groups from the $4^{th}$ week onwards was lower than that of the CE2EX group, and the food consumption of the CPL and CPLEX groups after 15 weeks was significantly lower than that of the CE2EX group.

Table 1:

| Effect of CPL on food consumption per day of obese mice (All results are shown as average±standard deviation) | | | |
|---|---|---|---|
| Feeding period Feeding period (weeks) | Food consumption per day (g/day) | | |
| | CE2EX | CPL | CPLEX |
| 1 | 4.94±0.46 | 5.31±1.35 | 5.21±0.56 |
| 4 | 6.98±0.76 | 6.78±0.74 | 6.57±0.75 |
| 10 | 6.15±0.64 | 5.59±0.81 | 5.91±1.12 |
| 15 | 5.87±0.95 | 4.27±1.33* | 4.57±0.98* |

*: When compared with the food consumption of the CE2EX group on the $15^{th}$ week, a significant difference was found in Student's t-test ($p<0.05$).

Test Example 2: Effect of CPL on increase of caloric release

There was a relation of inverse variation between the blood sugar level and. the caloric release of obese mice fed with CE2, and the higher the blood sugar level, the lower the level of caloric release. That is, since obese mice

that generate less heat and have lower caloric release are not very active in energy metabolism, they do not consume much sugar and lipid, and thereby have a high blood sugar level.

In a case where obese mice were fed with standard solid food CE2 containing 1% CPL and were subjected to swimming in the same manner as in Test Example 1 (CPLEX group) and a case where obese mice were fed with standard solid food CE2 (CE2 group), blood sugar level and caloric release were determined, and the results are shown in the following Table 2.

As is clear from the results shown in Table 2, even where blood sugar level is equal, caloric release in the CPLEX group is higher than that of the CE2 group. This result indicates that the basal metabolism of obese mice was increased by administration of CPL.

Table 2: Relation between blood sugar level and caloric release of obese mice

| Blood sugar level (mg/dl) | Caloric release (KJ) | |
|---|---|---|
| | CE2 | CPLEX |
| 180.2 | | 26 |
| 198.2 | | 39 |
| ditto | | 30 |
| 216.2 | 34 | |
| ditto | 26 | |
| 252.2 | 24 | |
| 288.2 | | 37 |
| ditto | | 33 |
| 324.2 | 18 | 37 |
| 342.3 | 14 | |
| 378.3 | 12.5 | |

Test Example 3: Effect of combined use of CPL and swimming on increase of lipid-using muscles

Rats were divided into two groups, and one group was fed with standard solid food CE2 (CE2 group; 12 mice) and another group was fed with standard solid food containing 0.1% CPL (CPL/swimming group; 12 mice). Rats were subjected to swimming for 10 minutes once every day in the first week. Then, rats were subjected to swimming for 20 minutes once every day in the second week, and for 30 minutes once every day from the third week onwards.

Seven days after feeding, body weight, and the weight of each organ, of the rats were determined. The results are shown in the following Table 3.

As is clear from the results shown in Table 3, the weight of the heart and the gastrocnemius increased due to the combination of CPL administration and swimming, but in contrast, the weight of fat tissues decreased. The heart and the gastrocnemius are muscles, which contains a large amount of mitochondria and actively use lipids. It has been found that the combined use of CPL and swimming increases muscles that actively use lipids and alters the body such that it uses lipids well, and thereby reduces the weight of fat tissues.

Table 3: Effect of CPL/swimming on relative value of the weight of organs and tissues
(All results are shown as average±standard deviation)

| | CE2 group | CPL/swimming group |
|---|---|---|
| Body weight (g) | 30.6±2.2 | 29.1±2.2 |
| Heart/Body weight (%) | 0.28±0.01 | 0.33±0.03* |
| Liver/Body weight (%) | 3.4±0.31 | 3.5±0.30 |
| Gastrocnemius/Body weight (%) | 0.52±0.04 | 0.58±0.06* |
| Soleus muscle/Body weight (%) | 0.46±0.05 | 0.48±0.06* |
| Fat tissues/Body weight (%) | 0.49±0.05 | 0.46±0.09* |

*: When compared with each of the values of the CE2 group, a significant difference was found in Student's t-test ($p<0.05$).

Test Example 4: Effect of the combined use of CPL and swimming on weight reduction of obese mice

Obese mice were divided into two groups: (1) a CE2EX group (12 mice), which were fed with standard solid

food CE2 and were subjected to swimming for 20 minutes every other day; and (2) a CPLEX group (12 mice), which were fed with standard solid food CE2 containing 1 weight % of CPL and were subjected to swimming for 20 minutes every other day, and then alteration of body weight (g) thereof was observed. Moreover, setting the average body weight of each group at the initiation point of the experiment at 100%, rates of increase were calculated (values in parentheses of Table 4). Since the body weight of a group in which mice were fed with standard solid food CE2 and were not subjected to swimming, was almost the same as the body weight of the CE2EX group, the data of this group was omitted. The results are shown in the following Table 4.

As is clear from the results shown in Table 4, the rates of increase of body weight of the CPLEX group was lower than that of the CE2EX group, and the body weight of the CPLEX group 15 weeks after initiation of the experiment was lighter than that of the CE2EX group.

During the experimental period, the average increase in body weight of the CE2EX group was 23.6g, whereas that of the CPLEX group was 18.0g. Weight increase was represented by about 23% by administration of CPL.

Table 4:

| Effect of CPL/swimming on body weight of obese mice<br>(All results are shown as average±standard deviation) | | |
|---|---|---|
| Feeding period (weeks) | CE2EX | CPLEX |
| 1 | 30.1±1.3(100) | 32.0±1.7(100) |
| 4 | 43.4±2.06(144) | 44.3±1.8(138) |
| 10 | 49.1±1.9(163) | 50.8±2.3(159) |
| 15 | 53.7±2.1(178) | 50.0±2.2(156)* |

* : A significant difference was found with the body weight of the CE2EX group in Student's t-test ($p<0.05$).

As is clear from the results of the above Test Examples 1 to 4, administration of CPL enabled a reduction of the food consumption of obese mice and promotion of the basal metabolism thereof. Furthermore, the combination of CPL administration and swimming enabled an increase in the muscle mass in gastrocnemius, cardiac muscle, etc. that actively use lipids, while not exerting an influence upon organs including the liver, and an increase in energy consumption, and reduced fat tissues by altering the body such that it uses lipids well, thereby reducing body weight.

CPL alone exhibited an action of repressing overeating or an action of promoting basal metabolism, and when CPL was combined with swimming, it clearly exhibited an action of improving obesity. Since no side effects are observed even where CPL is continuously administered in large amounts over a long period, CPL is useful for improvement of overeating or obesity of humans.

INDUSTRIAL APPLICABILITY

The agent or food and drink of the present invention is useful for suppression of appetite, promotion of basal metabolism, improvement and/or prevention of obesity, and enhancement of the effect of kinesitherapy. Moreover, a mixture of poly lactic acids used as an active ingredient in the present invention is a low condensate of lactic acids derived from organism components, and so it has a high biocompatibility with few side effects.

**Claims**

**1.** An appetite suppressing agent which comprises, as an active ingredient, a mixture of cyclic and/or straight chain poly lactic acids having a condensation degree of 3 to 19.

**2.** A basal metabolism promoting agent which comprises, as an active ingredient, a mixture of cyclic and/or straight chain poly lactic acids having a condensation degree of 3 to 19.

**3.** An agent for improving and/or preventing obesity which comprises, as an active ingredient, a mixture of cyclic and/or straight chain poly lactic acids having a condensation degree of 3 to 19.

**4.** An agent for enhancing the effect of kinesitherapy which comprises, as an active ingredient, a mixture of cyclic and/or straight chain poly lactic acids having a condensation degree of 3 to 19.

**5.** The agent according to any one of claims 1 to 4, wherein the lactic acid that is a repeating unit in the poly lactic

acid consists substantially of L-lactic acid.

6. The agent according to any one of claims 1 to 5, wherein the mixture of cyclic and/or straight chain poly lactic acids having a condensation degree of 3 to 19 is a fraction obtained by condensing lactic acids by dehydration under an inactive atmosphere, subjecting the ethanol- and methanol-soluble fractions of the obtained reaction solution to reverse phase column chromatography, and eluting with 25 to 50 weight % acetonitrile aqueous solution of pH 2 to 3 and then with 90 weight % or more acetonitrile aqueous solution of pH 2 to 3.

7. The agent according to claim 6, wherein condensation by dehydration is performed by stepwise decompression and temperature rise under nitrogen gas atmosphere.

8. The agent according to claim 6 or 7, wherein reverse phase column chromatography is performed by ODS column chromatography.

9. Food and drink for suppression of appetite, promotion of basal metabolism, improvement and/or prevention of obesity, and/or enhancement of the effect of kinesitherapy, which comprises, as an active ingredient, a mixture of cyclic and/or straight chain poly lactic acids having a condensation degree of 3 to 19.

Fig.1

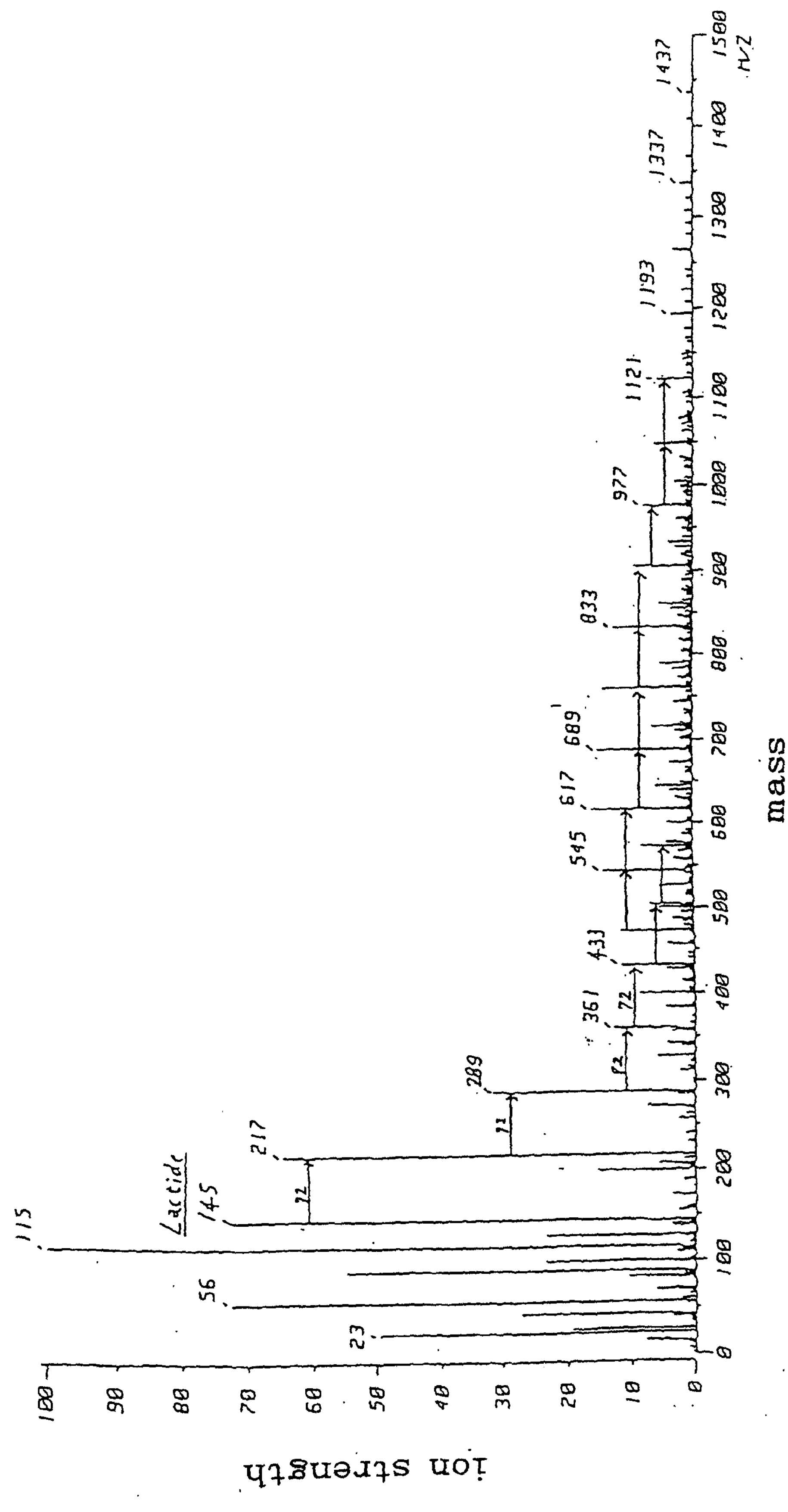
ion strength
mass
M/Z
Lactide
23
56
115
145
217
289
361
433
545
617
689
833
977
1121
1193
1337
1437
72
0
10
20
30
40
50
60
70
80
90
100
200
300
400
500
600
700
800
900
1000
1100
1200
1300
1400
1500

## INTERNATIONAL SEARCH REPORT

International application No.
PCT/JP00/06704

A. CLASSIFICATION OF SUBJECT MATTER
Int.Cl$^7$ A61K31/765, C07D323/00, A61P3/00, 3/04, 43/00

According to International Patent Classification (IPC) or to both national classification and IPC

B. FIELDS SEARCHED

Minimum documentation searched (classification system followed by classification symbols)
Int.Cl$^7$ A61K31/765, C07D323/00, A61P3/00, 3/04, 43/00

Documentation searched other than minimum documentation to the extent that such documents are included in the fields searched

| | | | |
|---|---|---|---|
| Jitsuyo Shinan Koho | 1926-1992 | Toroku Jitsuyo Shinan Koho | 1994-1996 |
| Kokai Jitsuyo Shinan Koho | 1971-1992 | Jitsuyo Shinan Toroku Koho | 1996-2000 |

Electronic data base consulted during the international search (name of data base and, where practicable, search terms used)
CA(STN),MEDLINE(STN),BIOSIS(STN),EMBASE(STN)

C. DOCUMENTS CONSIDERED TO BE RELEVANT

| Category* | Citation of document, with indication, where appropriate, of the relevant passages | Relevant to claim No. |
|---|---|---|
| A | US, 5527784, A (Kazuoki Isihara), 18 June, 1996 (18.06.96), Full text & JP, 5-339159, A | 1-9 |
| A | JP, 9-227388, A (Tetsuaki NAGANUSHI), 02 September, 1997 (02.09.97), Full text (Family: none) | 1-9 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 18 December, 2000 (18.12.00) | 26 December, 2000 (26.12.00) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP00/06704

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

The invention as set forth in claim 1 pertains to medicinal preparations wherein specific publicly known substances are used in regulating appetite. In contrast thereto, the invention as set forth in claim 2 and the invention as set forth in claim 4 pertain medicinal preparations wherein the above-described substances are used respectively for promoting the basal metabolism and potentiating the effects of exercise therapy. The invention as set forth in claim 1 has a technical feature of finding the use of the above-described substances for regulating appetite. However, the use of regulating appetite has no rational technical relationship to the use of promoting the basal metabolism. Also, the use of regulating appetite has no such relationship to the use of potentiating the effects of exercise therapy. Such being the case, it does not appear that there is a technical relationship between the invention of claim 1 and the inventions of claims 2 and 4 involving one or more of the same or corresponding special technical feature.

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)